# FASCICULE DE BREVET EUROPEEN

(11) **EP 0 597 032 B1**
(45) Date de publication et mention de la délivrance du brevet: **07.01.1998**
(21) Numéro de dépôt: 92917877.0
(22) Date de dépôt: 23.07.1992
(51) Int. Cl.: A61M 21/00, A61B 5/024

(54) **DISPOSITIF DE REVEIL BIOLOGIQUE PROGRAMMABLE EN FONCTION DES PHASES DU SOMMEIL**
ENTSPRECHEND DEN SCHLAFPHASEN PROGRAMMIERBARE VORRICHTUNG ZUM AUFWECKEN
BIOLOGICAL WAKE-UP DEVICE TO BE PROGRAMMED ACCORDING TO SLEEP EPISODES

(30) Priorité: 26.07.1991 FR 9109702
(43) Date de publication de la demande: 18.05.1994
(73) Titulaire: UNIVERSITE DE RENNES 1, 35000 Rennes (FR); CENTRE STEPHANOIS DE RECHERCHES MECANIQUES HYDROMECANIQUES ET FROTTEMENT (HEF), 42166 Andrezieux-Boutheon (FR)
(72) Inventeur: BILLON, Michel Rue Louis-Pasteur, F-22300 Lannion (FR); MARTIN, Eric, F-22700 Louannec (FR); CABASSE, Gilbert, F-29215 Guipavas (FR)
(74) Mandataire: Vidon, Patrice
(86) Numéro de dépôt international: FR9200723
(87) Numéro de publication internationale: WO9302731

(56) Documents cités:
- DE-A- 3 102 239
- FR-A- 2 634 913
- US-A- 4 052 979
- US-A- 4 101 071
- US-A- 4 509 531
- PATENT ABSTRACTS OF JAPAN vol. 012, no. 494 (P-805)23 Décembre 1988 & JP-A-63 205 592 ( SEIKO ) 25 Août 1988

## Description

Le domaine de l'invention est celui de la détection des différentes phases du sommeil chez un sujet. Plus précisément, l'invention concerne un dispositif permettant le réveil d'un sujet, en fonction des phases de son sommeil.

On sait qu'une nuit de sommeil est divisible en 4 à 6 cycles, dont la durée varie de 60 à 120 minutes selon les individus, et selon l'état de fatigue ou de stress de ces individus. Chacun de ces cycles peut être décomposé en trois types de phases: le sommeil lent léger, le sommeil lent profond et le sommeil paradoxal.

Le sommeil lent léger se retrouve plusieurs fois par cycle. Il s'agit essentiellement d'une phase de transition entre l'éveil et une autre phase, ou entre deux autres phases. Le phase de sommeil lent léger se caractérise par un tonus musculaire faible et un ralentissement cardiaque.

Le sommeil lent profond apparaît surtout lors du premier cycle. Sa durée diminue fortement lors des cycles suivants. C'est le sommeil de récupération. Sa durée dépend des efforts de la journée. Ainsi, après une privation de sommeil, sa durée par rapport aux autres phases passe de 20 % à 40 %. Le quantité de sommeil lent profond par nuit est invariante suivant le type de dormeur (petit ou grand dormeur). Durant cette phase, la mémorisation est renforcée, et les rêves sont généralement constitués d'éléments rationnels brefs.

L'éveil à partir du sommeil lent profond est suivi d'une phase plus ou moins longue de confusion accompagnée de désorientation et de défaillance de la mémoire.

Le sommeil paradoxal constitue la principale période de rêves. Le tonus musculaire est alors totalement aboli, et des mouvements oculaires rapides sont perceptibles. Les rêves lors de cette période sont longs, animés et plus étranges que lors du sommeil profond. Ils appartiennent davantage au domaine de l'affectivité.

Un réveil pendant cette période de sommeil peut provoquer un stress. Par ailleurs, le corps est alors engourdi.

S'il y a réveil juste après le sommeil paradoxal, le sujet est présent et lucide. Il se souvient de ses rêves.

Une augmentation de la durée de cette phase ne s'observe qu'après une privation de sommeil importante.

On constate donc que l'état du sujet, au réveil, dépend fortement de la phase de sommeil dans laquelle ce réveil se produit. Pour être agréable, le réveil doit avoir lieu lors d'une période de sommeil lent léger. Sinon, ou il y a confusion si le réveil a lieu lors du sommeil profond, ou bien il y a stress s'il y a interruption de rêve lors de la phase paradoxale.

Des dispositifs de détection des phases du sommeil sont déjà utilisés, dans le domaine médical. Il s'agit de dispositifs complexes, mettant en oeuvre plusieurs capteurs répartis sur le corps du patient, et reliés à une unité de traitement externe. Ces capteurs mesurent par exemple le tonus musculaire, le rythme cardiaque, le mouvement des yeux et la température du corps. Ces dispositifs, très précis car utilisés à des fins médicales, sont coûteux, et perturbent le sommeil, notamment lorsque le patient bouge.

On a également déjà réalisé des réveils fonctionnant en correspondance avec le rythme biologique d'un individu. Ainsi, le brevet français FR 86 06241, déposé au nom de Paul Boucheron, décrit un dispositif assurant le réveil d'un individu après une durée de sommeil correspondant à un nombre entier de cycles de sommeil. Ce dispositif, basé uniquement sur le calcul d'une durée optimale, s'avère non efficace, du fait que la durée des cycles du sommeil d'une personne n'est pas constante dans le temps.

Dans le certificat d'addition FR 88 10126, déposé le 27 juillet 1988 au nom du même déposant, il est proposé de pondérer le calcul de la durée optimale de sommeil calculée, en repérant la fin du dernier cycle de sommeil. Pour ce faire, on utilise un dispositif détectant l'apparition du sommeil paradoxal du sujet, en reconnaissant les mouvements des paupières et des yeux caractéristiques de cette phase. Deux électrodes sont pour cela disposées à la commissure des yeux, et une troisième est placée sur le corps du sujet, à titre de référence.

A nouveau, ce dispositif est à base de capteurs, ou électrodes, reliés à des moyens de traitements éloignés. L'inconfort de tels dispositifs apparaît clairement, et les limite à des applications médicales et temporaires. On imagine mal, en effet, qu'un particulier puisse accepter de se placer des électrodes à proimité des yeux, tous les soirs, pour s'assurer un bon réveil.

Par ailleurs, le dispositif décrit dans ce certificat d'addition est limité à la détection du sommeil paradoxal. Il ne permet pas de suivre les différentes phases du sommeil, ni d'assurer un réveil dans toute phase de sommeil, mais seulement à la fin du sommeil paradoxal.

Le brevet japonais JP-A-63205592 décrit un dispositif de détection de la phase de sommeil paradoxal mettant en oeuvre plusieurs électrodes réparties sur le corps du patient et reliées à une unité de traitement externe.

Ce dispositif comprend:
- deux capteurs d'activité cardiaque
- un microprocesseur qui permet de comparer les signaux des capteurs à ceux en mémoire et ainsi d'analyser toutes les phases du sommeil
- une alarme pour réveiller le sujet, activée par le microprocesseur en fonction des données recueillies au cours d'une phase du sommeil, lorsque certaines conditions sont réunies.

Comme déjà indiqué, ce type de dispositifs présentent de nombreux inconvénients. En particulier, ils sont coûteux et perturbent le sommeil des patients sur lesquels ils sont utilisés. La présence des électrodes fixées à différents endroits du corps du sujet peut en effet gêner celui-ci, par exemple lorsqu'il se retourne au cours de son sommeil.

De plus, ces dispositifs ne permettant qu'une simple mesure du rythme cardiaque, il peut conduire à une confusion entre le sommeil et l'éveil puisqu'il ne peut différencier un ralentissement du pouls durant l'éveil d'un ralentissement du pouls durant le sommeil paradoxal.

L'invention a notamment pour objectif de pallier ces inconvénients et limites de l'état de la technique.

Plus précisément, un objectif de l'invention est de fournir un dispositif de réveil programmable en fonction des phases du sommeil d'un sujet, qui soit non perturbateur pour le sommeil, même en cas de mouvements du sujet.

Un autre objectif de l'invention est de fournir un tel dispositif, permettant le réveil lors de toute phase de sommeil, de façon à, par exemple :
- assurer un réveil agréable en phase de sommeil lent léger;
- assurer un réveil en phase de sommeil paradoxal, pour prendre conscience de ses rêves;
- assurer un réveil après une ou plusieurs phases de sommeil lent profond, les plus réparatrices vis-à-vis de la fatigue.

L'invention a également pour objectif de fournir un tel dispositif qui soit relativement peu coûteux, et aisément industrialisable. Un objectif particulier de l'invention est en effet de fournir un tel dispositif pouvant être utilisé régulièrement, tous les jours, par toute personne souhaitant mieux gérer son sommeil.

Un autre objectif de l'invention est encore de fournir un tel dispositif, déterminant des informations sur le sommeil, telles que la durée de chaque phase, le nombre d'heures de sommeil sur une durée donnée,...

De façon complémentaire, l'invention a également pour objectif de fournir un tel dispositif, produisant des informations médicales sur le porteur du dispositif, telles que le rythme cardiaque, et pouvant générer des alertes en cas de problèmes.

Ces objectifs, ainsi que d'autres qui apparaîtront par la suite, sont atteints selon l'invention à l'aide d'un dispositif programmable de réveil biologique d'un sujet, réalisant l'intégration dans un même boîtier autonome portable:
- d'un capteur d'activité cardiaque ;
- de moyens de suivi de toutes les phases du sommeil, par analyse du signal relevé par ledit capteur ;
- de moyens de programmation d'un moment biologique de réveil dudit sujet, au cours de l'une quelconque desdites phases de sommeil ;
- de moyens de réveil dudit sujet en fonction dudit moment biologique de réveil programmé.

Le dispositif de l'invention comprend donc un capteur d'activité cardiaque, qui est apparu être l'information la plus efficace pour déterminer précisément les différentes phases du sommeil sans nécessiter d'appareillage important.

En effet, l'invention ne vise pas à fournir un système ayant la précision nécessaire au domaine médical, mais un dispositif simple et pratique "d'assistance au réveil". Ainsi, le dispositif de l'invention intègre toutes ces fonctions, à savoir notamment la détermination de chaque phase du sommeil et le réveil dans l'une de ces phases, en fonction de la programmation choisie, dans un seul boîtier autonome, et ne nécessite pas, par exemple, l'usage de diverses électrodes extérieures au boîtier.

Avantageusement, ledit boitier est susceptible d'être fixé au poignet dudit sujet à l'aide d'un bracelet.

En effet, le bracelet-montre semble être l'objet le plus pratique : son usage est entré dans les moeurs, il ne perturbe pas le sommeil, et permet une programmation relativement aisée, du même type que celle réalisable sur les montres-réveils actuelles.

Dans un mode de réalisation particulier de l'invention, le dispositif comprend également un capteur de la température du corps et/ou un capteur du tonus musculaire du sujet et/ou un capteur du débit sanguin.

Cela permet d'obtenir une plus grande précision encore sur la détermination des phases du sommeil. Ces capteurs supplémentaires ne sont toutefois absolument pas obligatoires pour le bon fonctionnement de l'invention.

De façon avantageuse, ledit capteur d'activité cardiaque comprend un capteur de pression, réagissant au pouls dudit sujet.

Préférentiellement, ledit capteur de pression comprend un élément de céramique piézo-électrique, réagissant en flexion et/ou en traction/compression aux déplacements d'un capteur-plaque en contact direct avec le corps dudit sujet, et produisant entre ses deux faces une différence de potentiel proportionnelle auxdits déplacements.

Ce mode de réalisation, relativement simple à mettre en oeuvre même dans un espace restreint tel que celui d'une montre, permet d'obtenir une bonne précision sur le rythme cardiaque du sujet.

Avantageusement, ledit capteur d'activité cardiaque comprend un second capteur-plaque de compensation, isolé du corps dudit sujet et connecté à un second élément de céramique piézo-électrique, de façon à détecter les mouvements parasites dudit sujet.

Il est en effet important de discerner les variations de pression dues réellement au sang circulant dans les veines de celles provoquées par les mouvements du dormeur. Le second capteur ne détectant que ces mouvements parasites, il est alors possible de prendre en compte le signal issu de ce second élément pour améliorer la mesure de l'activité cardiaque.

Dans un mode de réalisation préférentiel de l'invention, ledit second capteur-plaque de compensation est placé au niveau de la connexion dudit bracelet sur ledit boitier.

De cette façon, ce second capteur détecte mieux les variations dues aux mouvements des tendons dans le poignet, lorsque le sujet remue les doigts.

Avantageusement, le dispositif de l'invention comprend des moyens d'analyse du signal produit par ledit second élément de céramique piézo-électrique, de façon à distinguer l'éveil du sommeil paradoxal.

De façon préférentielle, le dispositif comprend des moyens de traitement d'un signal relevé par ledit ou lesdits capteurs, comprenant :
- des moyens d'amplification du signal mesuré ;
- des moyens de filtrage du signal amplifié ;
- des moyens de numérisation du signal filtré ;
- des moyens d'analyse du signal numérisé ;
- des moyens de gestion du temps ;
- des moyens de commande desdits moyens de réveil, en fonction de ladite analyse et des données programmées.

Avantageusement, lesdits moyens d'analyse déterminent et/ou affichent au moins une des informations suivantes:
- début et fin de chaque phase et/ou cycle de sommeil ;
- durée de chaque phase et/ou cycle de sommeil ;
- durée probable du sommeil ou heure probable de réveil ;
- statistiques sur le sommeil, telles que rapports temps de veille/temps de sommeil ;
- effort cardiaque ;
- nombre de cycles de sommeil nécessaires pour une bonne récupération physique dudit sujet ;
- nombre de calories alimentaires nécessaires pour une bonne récupération physique dudit sujet ;
- alerte en cas de dépassement du rythme cardiaque maximum ou minimum toléré pour ledit sujet ;
- détection de maladies ou de disfonctionnements, notamment cardio-vasculaires, tels que l'ischémie.

D'autres types d'informations peuvent également être déterminées, notamment lorsque l'on prend en compte la température du corps ou le tonus musculaire.

De façon avantageuse, le dispositif selon l'invention comprend des moyens de communication avec des dispositifs externes, par exemple pour le transfert d'informations vers un micro-ordinateur et/ou pour la télécommande d'appareils.

D'autres caractéristiques et avantages de l'invention apparaîtront à la lecture de la description suivante d'un mode de réalisation préférentiel de l'invention, donnée à titre illustratif et non limitatif, et des dessins annexés, dans lesquels :
- la figure 1 est un schéma synoptique d'un mode de réalisation du dispositif de l'invention ;
- la figure 2 est un exemple de capteur pouvant être utilisé dans le dispositif de la figure 1, à base d'éléments céramiques piézo-électriques.

L'invention concerne donc un dispositif autonome et portable de détection des différentes phases du sommeil. Le mode de réalisation décrit ci-dessous en détail présente un tel dispositif prévu pour être placé dans un bracelet-montre.

Il apparaît en effet que le bracelet-montre est la forme la plus habituelle pour porter un engin électronique directement sur soi, et qu'il n'est pas gênant, même lorqu'on le porte la nuit. Il permet en outre une bonne présentation des informations collectées et une ergonomie satisfaisante. Par ailleurs, il n'impose pas le port d'un objet supplémentaire : le dispositif de l'invention peut également donner l'heure...

L'invention peut toutefois être placée dans d'autres types d'objets portables, tels que, en particulier, bagues, colliers, ou tous autres dispositifs maintenus par exemple par adhésifs, notamment à proximité du coeur.

A partir de la détermination des différents cycles, un circuit de décision déclenche une sonnerie pour réveiller l'utilisateur à une période propice. D'après les médecins, seul l'éveil pendant une phase de sommeil lent léger permet une prise de contact immédiate avec la réalité, sans stress. Il est à noter que ces périodes se présentent de plus en plus souvent en fin de sommeil, ce qui permet de prévoir une fourchette de réveil inférieure à un quart d'heure. De plus, le dispositif peut mémoriser une heure limite de réveil, ce qui permet d'éviter d'être en retard.

La détermination des phases du sommeil peut se faire notamment par surveillance du rythme cardiaque, de la température interne ou du tonus interne. Pour une plus grande précision, il est bien sûr possible de considérer plusieurs de ces paramètres en combinaison.

La mesure la plus fiable pour déterminer les cycles apparaît être le rythme cardiaque, dont l'analyse permet de détecter les phases régulières (sommeil profond) et les phases instables (sommeil paradoxal). Plus précisément, on sait que:
- l'entrée en sommeil lent induit une discrète bradycardie (ralentissement cardiaque) ;
- en sommeil lent profond on assiste à une stabilisation maximale des fonctions végétatives (fréquences cardiaques et respiratoires...) ;
- durant le sommeil paradoxal, il y a alternance de tachycardie et de bradycardie.

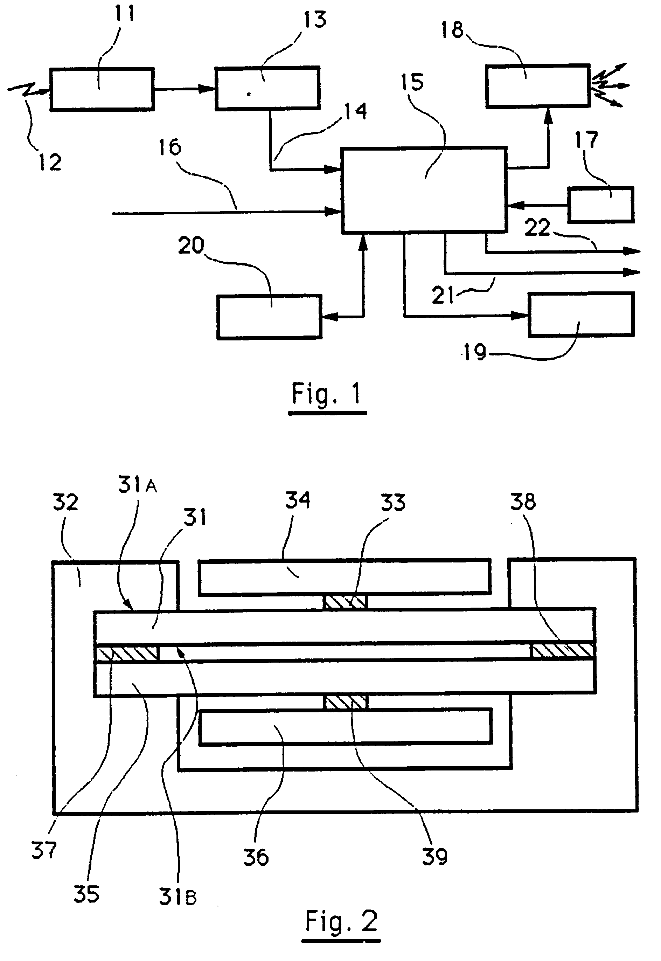

La figure 1 est un schéma synoptique d'un dispositif selon l'invention.

Un capteur 11 détecte les variations de rythme cardiaque 12, au niveau du poignet (dans le cas d'un bracelet), et éventuellement la température du corps et/ou le tonus musculaire. Un exemple de capteur utilisable est décrit plus loin, en relation avec la figure 2.

Le signal détecté par le capteur 11 est transmis à un module 13 de traitement du signal, qui assure notamment :
- l'amplification du signal, par exemple à l'aide d'un amplificateur à charge;
- le filtrage du signal, à l'aide d'un filtre passe-bas qui élimine le bruit haute fréquence induit par le secteur et les pointes de pressions dues aux mouvements parasites de bras ;
- la conversion analogique/numérique.

Le signal numérisé 14, échantillonné par exemple toutes les 20 millisecondes, est transmis à un module 15 de traitement, qui est par exemple un micro-contrôleur. Ce module 15 est chargé de faire l'acquisition et la détection du pouls, d'assurer le réveil du sujet en fonction de données 16 de programmation et des informations fournies par l'horloge 17, et éventuellement de déterminer les différents calculs annexes détaillés plus loin.

La détection du pouls comprend notamment les tâches suivantes :
- commande d'acquisition afin d'obtenir un échantillon de mesure toutes les N millisecondes (par exemple N=20);
- constitution d'un tableau de mesures ;
- calcul du logarithme de ces mesures pour réduire la dynamique du signal;
- calcul de l'autocorrélation de la courbe ainsi obtenue ;
- détection de périodicité sur l'autocorrélation ;
- déduction à partir de cette périodicité du pouls instantané ;
- constitution d'un tableau des mesures de pouls associées à l'heure de la mesure.

Lorsque le réveil du sujet est souhaitable, par exemple parce qu'il est dans une phase de sommeil lent léger à proximité de l'heure de réveil pré-programmée, le module 15 de traitement active un module 18 de réveil, comprenant par exemple une sonnerie.

Le module 15 de traitement est également relié à des moyens 19 de visualisation, tel qu'un afficheur, chargé d'indiquer l'heure, les informations programmées, des statistiques,...

Le dispositif selon l'invention comprend encore avantageusement des moyens 20 de stockage des mesures effectuées, pour permettre la réalisation de statistiques, ou simplement pour conserver suffisamment d'échantillons pour que la reconnaissance des phases soit fiable.

On peut encore prévoir des moyens de dialogue avec un dispositif externe, pour que des données 21 puissent être exploitées plus en profondeur. Il est ainsi possible de connecter l'invention à un micro-ordinateur, ou par l'intermédiaire d'un modem, de transmettre des informations régulières à un médecin, par exemple dans le cas de malades cardiaques.

Le dispositif de l'invention peut également transmettre des commandes 22 vers de nombreux types d'appareils (sonnerie, radio-réveil, appareil enregistreur, etc...), par câble, ou par télécommande à distance (infra-rouge par exemple).

La programmation de l'invention peut se faire de façon simple et adaptée aux besoins des utilisateurs. A titre d'exemple, il est possible de choisir :
- un réveil agréable : celui-ci doit avoir lieu lors d'une période de sommeil lent léger, sinon il y a confusion si le réveil a lieu lors du sommeil profond, ou stress s'il y a interruption de rêve lors de la phase paradoxale.
- un sommeil écourté à sa phase la plus importante pour la récupération, le sommeil profond : l'invention permet alors de se réveiller dès la fin de la première (et la plus longue) apparition de cette phase. Cela peut être extrêmement avantageux pour ceux pour qui le sommeil est une perte de temps : lors d'une surcharge momentanée de travail (surcharge professionnelle, examens...), pour des veilles prolongées (navigateurs, militaires...), pour les sportifs lors d'exploits d'endurance, etc...

Il est nécessaire de noter que cette limitation de la durée du sommeil n'est pas sans inconvénients lors d'une pratique systématique. Ce domaine de la physiologie fait actuellement le sujet de nombreuses études. Les résultats qu'apporteront celles-ci n'influenceront pas le mode de fonctionnement de l'invention présentée. Ils permettront cependant d'en obtenir une utilisation optimale.
- une prise de conscience des rêves : beaucoup de gens sont persuadés qu'ils rêvent très peu, et pas toutes les nuits, alors que le rêve occupe au minimum deux heures d'une nuit normale. Cette impression est due au fait que l'on se souvient rarement de ses rêves. La prise de conscience de ceux-ci peut notamment permettre :
   - une meilleure connaissance de soi,
   - une évasion et un étonnement sur sa propre créativité et imagination,
   - une utilisation de cet imaginaire à des fins créatrices et artistiques,
   - une prise de conscience de ses obsessions et une analyse de celles-ci (psychothérapie ou autothérapie).

La figure 2 représente un exemple de capteur d'activité cardiaque qui peut avantageusement être utilisé dans le dispositif décrit ci-dessus.

Il s'agit d'un capteur piézo-électrique captant le bruit, c'est-à-dire les changements de pression, provoqué par le passage rythmé du sang dans les veines, par exemple au niveau du poignet.

Un élément 31 en céramique piézo-électrique est maintenu dans un boîtier 32, par exemple en aluminium. Cet élément 31 a par exemple une taille de 12 x 4 x 0,7 mm. Une cale 33 est fixée, par collage, au centre de l'élément 31. Sur la cale 33 prend place une plaque 34 en aluminium, ou en tout autre matériau.

Cette plaque 34, qui peut avoir une surface de 7 x 7 mm, se trouve au contact de la peau du sujet, lorsque le dispositif est en place. La large surface de contact permet d'obtenir un signal de mouvement de forte puissance.

Ce mouvement est transmis à l'élément 31, par l'intermédiaire de la cale 33. Cela induit une flexion de l'élément, faisant apparaître une différence de potentiel entre les deux faces 31_{A} et 31_{B} de l'élément 31. C'est cette différence de potentiel qui est fournir au module 13 de traitement du signal de la figure 1.

Avantageusement, le capteur comporte un second groupe élément piézo-électrique 35 et plaque 36, isolé du corps. Ainsi la plaque libre 36 fournit un signal reflétant les mouvements du bras, mouvements qui induisent des signaux non utiles dans le premier capteur. Une réduction de ces parasites peut être obtenue en combinant les signaux par les deux éléments 31 et 35.

Les deux éléments piézo-électriques 31 et 35 sont isolés par deux cales 37 et 38. La cale 33, de même que la cale 39 séparant le second élément 35 et la seconde plaque 36, peuvent avoir 1 mm de large.

Le second groupe, ou "capteur de bruit parasite", peut également être positionné de façon différente, au niveau de la fixation du bracelet sur le boîtier. La céramique travaille alors en traction-compression, et non plus en flexion, et capte ainsi mieux les perturbations produites par les variations de pression sur le premier capteur (toujours appliqué sur la peau) tout en étant isolé du signal utile (rythme cardiaque). En effet, le capteur de bruit dans la solution précédente n'est pas sensible aux variations de tension du bracelet lors des modifications du diamètre du poignet provoquées par les mouvements des tendons. Cette solution assure donc une meilleure immunité vis-à-vis de l'activité des doigts.

Un tel dispositif, utilisant un capteur piézo-électrique, permet de détecter sans difficulté les trois types de phases de sommeil, pour un coût relativement faible.

Les périodes d'éveil et les périodes de sommeil paradoxal présentent des signaux de rythme cardiaques ayant des variabilités assez proches. Il est toutefois relativement aisé de les distinguer, en analysant le signal de bruit fourni par le second capteur. En effet, les périodes d'éveil produisent des séries de mesures présentant des variances supérieures à la normale, que l'on ne retrouve pas dans le cas du sommeil paradoxal.

De nombreuses applications de cette invention peuvent être envisagées. Ainsi, à titre d'exemple, elle est susceptible d'intéresser :
- les médecins hypnologues (traitant des troubles du sommeil) pour les problèmes de leurs patients, qui ne peuvent tenir un compte de leurs heures de sommeil. Une ou plusieurs nuits à l'hôpital sont jusqu'ici nécessaire pour recueillir des données scientifiques. Cela coûte évidemment très cher pour une fiabilité biaisé, car on sort le patient de son environnement, ce qui perturbe fortement son sommeil ;
- les médecins cardiologues, pour suivre l'effort cardiaque de leurs patients tout au long de la journée ;
- les particuliers ayant des surcharges de travail pendant quelques jours, et voudraient ne pas perdre de temps à rêver (étudiants, professions libérales, militaires ou navigateurs...) ;
- les malades cardiaques, qui doivent limiter leurs efforts. L'invention peut signaler un dépassement de seuil ou de fatigue accumulée ;
- les personnes souhaitant fractionner leurs temps de sommeil et faire une sieste, et voulant limiter la sieste au cycle le plus important et avoir une sieste courte et un réveil rapide ;
- les personnes ayant du mal à se lever !
- les personnes portant intérêt à leurs rêves ;
- les personnes qui veulent pouvoir gérer au mieux leurs efforts, leur récupération et leur alimentation ;
- etc...

Le dispositif de l'invention peut par ailleurs intégrer de nombreuses fonctions annexes, telles que :
- l'affichage de la durée des différents cycles,
- l'affichage de la quantité de sommeil et du rapport temps de veille/temps de sommeil sur diverses périodes (par exemple sur une semaine),
- l'extrapolation de l'effort fourni dans la journée par analyse de l'effort cardiaque,
- le calcul du nombre de cycles nécessaires pour une bonne récupération en fonction de l'effort fourni et des retards accumulés,
- l'évaluation du nombre de calories nécessaires à ingérer en fonction des mêmes critères (et du poids de l'usager),
- la mémorisation du maximum (et du minimum) cardiaque toléré et alarme en cas de dépassement, pour les sportifs et les malades du coeur,
- transmission par liaison série des différentes mesures vers un ordinateur (éventuellement via un modem), pour des nouvelles analyses et mémorisations. Cette fonction est particulièrement destinée aux différentes branches médicales qui ont divers besoins d'étude sur le coeur mais ne disposent pas de capteur enregistreur directement utilisable par tout-un-chacun. Cela concerne par exemple les médecins hypnologues, et les médecins sportifs qui pourront suivre le rythme de vie et mesurer les évolutions de la réaction à l'effort sans faire appel à d'encombrantes électrodes.
- la détection des anomalies cardiaques : arythmie, extrasystoles, parasystolie, échappements, tachycardie paroxystique, flutter, blocs, et surtout ischémie (signe avant coureur de l'infarctus).

Dans d'autres modes de réalisation de l'invention, prenant en compte par exemple la température interne et/ou le tonus musculaire, d'autres fonctions et calculs annexes pourront également être mis en oeuvre.

Plus généralement, de nombreuses adaptations et développements de l'invention sont envisageables, tant en ce qui concerne les types du ou des capteurs utilisés, les supports de l'invention (bracelets, bagues, systèmes adhésifs, etc...) ou les types de traitements et/ou de calculs effectués à partir des signaux issus des capteurs.

## Revendications

1. Dispositif programmable de réveil biologique d'un sujet, caractérisé en ce qu'il réalise l'intégration dans un même boîtier autonome portable:
- d'un capteur (11) d'activité cardiaque (12) comprenant, un capteur de pression, réagissant au pouls dudit sujet, ledit capteur de pression comprenant un élément (31) de céramique piézo-électrique, réagissant en flexion et/ou en traction/compression aux déplacements d'un premier capteur-plaque (34) en contact direct avec le corps dudit sujet, et produisant entre ses deux faces (31_{A}, 31_{B}) une différence de potentiel fonction desdits déplacements, ledit capteur d'activité cardiaque comprenant par ailleurs un second capteur-plaque (36) de compensation, isolé du corps dudit sujet et connecté à un second élément (35) de céramique piézo-électrique, de façon à détecter les mouvements parasites dudit sujet,
- de moyens (15) de suivi de toutes les phases du sommeil, par analyse du signal (14) relevé par ledit capteur (11) ;
- de moyens (15) de programmation d'un moment biologique (16) de réveil dudit sujet, au cours de l'une quelconque desdites phases de sommeil ;
- de moyens (15) d'analyse du signal produit par ledit second élément (35) de céramique piézo-électrique, de façon à distinguer l'éveil du sommeil paradoxal,
- de moyens (18) de réveil dudit sujet en fonction dudit moment biologique (16) de réveil programmé.

2. Dispositif selon la revendication 1, caractérisé en ce que ledit boitier est susceptible d'être fixé au poignet dudit sujet à l'aide d'un bracelet.

3. Dispositif selon l'une quelconque des revendications 1 ou 2, caractérisé en ce qu'il comprend également un capteur de la température du corps et/ou un capteur du tonus musculaire du sujet et/ou un capteur de débit sanguin.

4. Dispositif selon les revendications 1 à 3, caractérisé en ce que ledit second capteur-plaque (36) de compensation est placé au niveau de la connexion dudit bracelet sur ledit boitier.

5. Dispositif selon l'une quelconque des revendications 1 à 4, caractérisé en ce qu'il comprend des moyens de traitement d'un signal relevé par ledit ou lesdits capteurs (11), comprenant :
- des moyens (13) d'amplification du signal mesuré ;
- des moyens (13) de filtrage du signal amplifié ;
- des moyens (13) de numérisation du signal filtré ;
- des moyens (20) d'analyse du signal numérisé ;
- des moyens (17) de gestion du temps ;
- des moyens (15) de commande desdits moyens (18) de réveil, en fonction de ladite analyse et des données programmées (16).

6. Dispositif selon l'une quelconque des revendications 1 à 5, caractérisé en ce que lesdits moyens d'analyse (15) déterminent et/ou affichent (19) au moins une des informations suivantes:
- début et fin de chaque phase et/ou cycle de sommeil ;
- durée de chaque phase et/ou cycle de sommeil ;
- durée probable du sommeil ou heure probable de réveil ;
- statistiques sur le sommeil, telles que rapports temps de veille/temps de sommeil ;
- effort cardiaque ;
- nombre de cycles de sommeil nécessaires pour une bonne récupération physique dudit sujet ;
- nombre de calories alimentaires nécessaires pour une bonne récupération physique dudit sujet ;
- alerte en cas de dépassement du rythme cardiaque maximum ou minimum toléré pour ledit sujet ;
- détection de maladies ou de disfonctionnements, notamment cardio-vasculaires, tels que l'ischémie.

7. Dispositif selon l'une quelconque des revendications 1 à 6, caractérisé en ce que ledit capteur (11) d'activité cardiaque est en contact direct avec le corps dudit sujet.

8. Dispositif selon l'une quelconque des revendications 1 à 7, caractérisé en ce qu'il comprend des moyens de communication (21,22) avec des dispositifs externes.

## Claims

1. Programable device for the biological wakening of a subject, characterized in that there is integrated in the same self-contained control box:
- a sensor (11) of cardiac activity (12) comprising a pressure sensor, reacting to the pulse of the said subject, the said pressure sensor comprising a piezo-electric ceramic element (31), reacting in deflexion and/or in tension/compression to the movements of a first plate-sensor (34) in direct contact with the body of the said subject, producing between its two faces (31_{A},31_{B}) a difference in potential as a function of the said movements, the said sensor of cardiac activity additionally comprising a second compensating plate-sensor (36), insulated from the body of the said subject and connected to a second piezo-electric ceramic element (35), so as to detect interfering movements of the said subject,
- means (15) of following all the phases of sleep, by analysing the signal (14) detected by the said sensor (11);
- means (15) for programming a biological moment (16) of wakening of the said subject, during any one of the said phases of sleep;
- means (15) for analysing the signal produced by the said second piezo-electric ceramic element (35), so as to distinguish wakening from paradoxical sleep,
- means (18) for wakening the said subject as a function of the said programmed biological moment (16) of wakening.

2. Device according to claim 1, characterized in that the said control box can be attached to the wrist of the said subject with the aid of a bracelet.

3. Device according to either of claims 1 or 2, characterized in that it also comprises a body temperature sensor and/or a sensor of the muscular tonus of the subject and/or a blood flow sensor.

4. Device according to claims 1 to 3, characterized in that the said second compensating plate-sensor (36) is placed at the level of the connection of the said bracelet to the said control box.

5. Device according to any one of claims 1 to 4, characterized in that it comprises means for processing a signal detected by the said sensor or sensors (11), comprising :
- means (13) for amplifying the signal measured;
- means (13) for filtering the amplified signal;
- means (13) for digitizing the filtered signal;
- means (20) for analysing the digitized signal;
- means (17) for time management;
- means (15) for controlling the said means (18) of wakening, as a function of the said analysis and the programmed data (16).

6. Device according to any of claims 1 to 5, characterized in that the said means of analysis (15) determine and/or display (19) at least one of the following pieces of information :
- start and finish of each sleep phase and/or cycle;
- duration of each sleep phase and/or cycle;
- probable duration of sleep or the probable time of wakening;
- statistics on sleep, such as the ratios of times of wakening/times of sleep;
- cardiac effort;
- number of sleep cycles necessary for the good physical recovery of the said subject;
- number of food calories necessary for the good physical recovery of the said subject;
- warning in the case of a departure from the maximum or minimum cardiac rhythm tolerated by the said subject;
- detection of diseases or malfunctions, in particular cardio-vascular diseases or malfunctions, such as ischaemia.

7. Device according to any of claims 1 to 6, characterized in that the said sensor (11) of cardiac activity is in direct contact with the body of the said subject.

8. Device according to any of claims 1 to 7, characterized in that it comprises means of communication (21,22) with the external devices.

## Patentansprüche

1. Programmierbare Vorrichtung zum biologischen Aufwecken eines Menschen,
dadurch gekennzeichnet, daß in einem einzigen, unabhängig tragbaren Gehäuse integriert sind:
- ein Meßaufnehmer (11) für die Herzaktivität (12) umfassend einen Druckaufnehmer, der auf den Puls des Menschen reagiert, wobei der Druckaufnehmer ein keramisches, piezo-elektrisches Element (31) umfaßt, das auf Biegung und/oder auf Zug bzw. Druck bei Ortsveränderungen einer sich in direktem Kontakt mit dem Körper des Menschen befindlichen ersten Aufnehmerplatte (34) reagiert und zwischen seinen beiden Flächen (31_{A}, 31_{B}) eine Potentialdifferenz als Funktion dieser Ortsveränderungen erzeugt, wobei der Aufnehmer für die Herzaktivität weiterhin eine zweite Aufnehmerplatte (36) zur Kompensation umfaßt, welche vom Körper isoliert ist und mit einem zweiten keramischen, piezo-elektrischen Element (35) verbunden ist, um Störbewegungen des Menschen zu detektieren,
- Mittel (15) zur Verfolgung aller Schlafphasen durch Analyse des vom Aufnehmer (11) aufgenommenen Signals (141);
- Mittel (15) zur Programmierung eines biologischen Aufweckmomentes (16) des Menschen während einer der Schlafphasen;
- Mittel (15) zur Analyse des vom zweiten keramischen, piezo-elektrischen Elements (35) erzeugten Signals, um das Erwachen aus paradoxem Schlaf zu unterscheiden,
- Mittel (18) zum Aufwecken des Menschen als Funktion des programmierten biologischen Aufweckmomentes (16).

2. Vorrichtung nach Anspruch 1,
dadurch gekennzeichnet, daß das Gehäuse am Handgelenk des Menschen mit Hilfe eines Armbandes fixierbar ist.

3. Vorrichtung nach Anspruch 1 oder 2,
dadurch gekennzeichnet, daß es ebenfalls einen Aufnehmer für die Temperatur des Körpers und/oder einen Aufnehmer für den Muskeltonus des Menschen und/oder einen Aufnehmer für den Blutfluß umfaßt.

4. Vorrichtung nach des Ansprüchen 1 bis 3,
dadurch gekennzeichnet, daß die zweite Aufnehmerplatte (36) zur Kompensation auf Höhe der Verbindung des Armbandes mit dem Gehäuse angeordnet ist.

5. Vorrichtung nach einem der Ansprüche 1 bis 4,
dadurch gekennzeichnet, daß sie Mittel zur Verarbeitung des aufgenommenen Signals von dem oder den Aufnehmern (11) umfaßt, welche umfassen:
- Mittel (13) zur Verstärkung des gemessenen Signals;
- Mittel (13) zur Filterung des verstärkten Signals;
- Mittel (13) zur Digitalisierung des gefilterten Signals;
- Mittel (20) zur Analyse des digitalisierten Signals;
- Mittel (17) zur Zeitsteuerung;
- Mittel (15) zum Auslösen der Mittel (18) zum Aufwecken, als Funktion der Analyse und der programmierten Daten (16).

6. Vorrichtung nach einem der Ansprüche 1 bis 5,
dadurch gekennzeichnet, daß die Mittel zur Analyse (15) mindestens eine der folgenden Informationen bestimmen und/oder anzeigen (19):
- Anfang und Ende jeder Schlafphase und/oder jedes Schlafzyklusses;
- Dauer jeder Schlafphase und/oder jedes Schlafzyklusses;
- wahrscheinliche Schlafdauer oder wahrscheinliche Aufweckzeit;
- Schlafstatistiken mit Wiedergabe der Wachzeiten/Schlafzeiten;
- Herztätigkeit;
- Anzahl der notwendigen Schlafzyklen für eine gute physische Erholung des Menschen;
- Anzahl der notwendigen Kalorien in der Nahrung für eine gute physische Erholung des Menschen;
- Warnung im Falle des Überschreitens des Herzrhythmusses von einem für den Menschen tolerierbaren Maximum oder Minimum;
- Detektion von Krankheiten oder Disfunktionen, insbesondere cardiovasculären, wie beispielsweise Ischämie.

7. Vorrichtung nach einem der Ansprüche 1 bis 6,
dadurch gekennzeichnet, daß der Aufnehmer (11) der Herzaktivität in direktem Kontakt mit dem Körper des Menschen ist.

8. Vorrichtung nach einem der Ansprüche 1 bis 7,
dadurch gekennzeichnet, daß sie Mittel zur Kommunikation (21, 22) mit externen Vorrichtungen umfaßt.
